# EUROPEAN PATENT SPECIFICATION

(11) **EP 3 536 153 B1**
(45) Date of publication and mention of the grant of the patent: **23.12.2020**
(21) Application number: 19161182.1
(22) Date of filing: 07.03.2019
(51) Int. Cl.: A01N 63/25, A01N 63/22, A01N 63/27, A01N 63/20, A01P 1/00, A01P 21/00, A01P 3/00

(54) **THE COMPOSITION COMPRISING ISOLATED STRAINS OF SAPROPHYTIC SOIL BACTERIA, BIOPREPARATION CONTAINING SUCH COMPOSITION AND METHODS AND USES THEREOF**
DIE ZUSAMMENSETZUNG ENTHALTEND ISOLIERTE STÄMME VON SAPROPHYTISCHEN BODENBAKTERIEN, BIOPREPARATION, ENTHALTEND DIESE ZUSAMMENSETZUNG UND VERFAHREN SOWIE IHRE VERWENDUNGEN
COMPOSITION COMPRENANT DES SOUCHES ISOLEES DE BACTERIES DU SOL SAPROPHYTIQUES, BIOPREPARATION CONTENANT UNE TELLE COMPOSITION, PROCEDES ET UTILISATIONS ASSOCIES

(30) Priority: 08.03.2018 PL 42479418
(43) Date of publication of application: 11.09.2019
(73) Proprietor: BACTrem Sp. z o.o., 02-096 Warszawa (PL)
(72) Inventor: POPOWSKA, Magdalena, 05-500 Nowa Iwiczna (PL)
(74) Representative: Grzelak, Anna

(56) References cited:
- WO-A1-2016/178086
- US-A1- 2016 374 341
- JAFRA S ET AL: "Potential of bulb-associated bacteria for biocontrol of hyacinth soft rot caused by Dickeya zeae", JOURNAL OF APPLIED MICROBIOLOGY, WILEY-BLACKWELL PUBLISHING LTD, GB , vol. 106, no. 1 1 January 2009 (2009-01-01), pages 268-277, XP002671554, ISSN: 1364-5072, DOI: 10.1111/J.1365-2672.2008.04000.X Retrieved from the Internet: URL:http://onlinelibrary.wiley.com/doi/10. 1111/j.1365-2672.2008.04000.x/abstract [retrieved on 2008-12-01]
- MILVA PEPI ET AL: "Two naphthalene degrading bacteria belonging to the genera Paenibacillus and Pseudomonas isolated from a highly polluted lagoon perform different sensitivities to the organic and heavy metal contaminants", EXTREMOPHILES ; LIFE UNDER EXTREME CONDITIONS, SPRINGER-VERLAG, TO, vol. 13, no. 5, 21 July 2009 (2009-07-21), pages 839-848, XP019742658, ISSN: 1433-4909, DOI: 10.1007/S00792-009-0271-1

## Description

### TECHNICAL FIELD

The object of the invention is a composition comprising 8 strains of saprophytic soil bacteria representing the genera: *Arthrobacter, Bacillus, Citrobacter, Peanibacillus, Pseudomonas,* which contains the isolated strains *Arthrobacter* sp. strain 1.4, *Bacillus* sp. strain 202B, *Bacillus* sp. strain 233, *Bacillus* sp. strain 307A, *Citrobacter* sp. strain 6A, *Peanibacillus* sp. strain 45, *Pseudomonas* sp. strain 23 (W), *Pseudomonas* sp. strain 60 (W), as well as a biopreparation containing such composition, and the method of production thereof. The object of the invention are also the methods of plant pathogen control in soil, soil fertilization and restoring the natural biological balance of soil microflora, biostimulation of plant development and growth, as well as uses for plant pathogen control, soil fertilization and restoring the natural biological balance of soil microflora, biostimulation of plant development and growth, using the composition and biopreparation comprising these 8 cooperating strains

### BACKGROUND ART

Plant pathogens, which use photosynthesis products produced by plants, constitute one of the biotic factors that cause their diseases. The action of pathogens is an important, undesirable aspect of the production of food plants. The effects of their activity range from mild symptoms to the destruction on entire areas of cultivation. Such extensive plant diseases can aggravate the global food deficit. The management of plant pathogens is hampered by the fact that they are very difficult to control. Their frequent transmission from diseased plants to healthy ones occurs, due to which their range expands over time. An example of a plant pathogen is *Psudomonas syringae,* a Gram-negative bacterium, situated on the first place of the list of the most dangerous bacterial plant pathogens (phytopathogens). More than 50 strains of this species have already been described, each of which is a pathogen of a specific plant species. This broad range includes both large and small crops. *P. syringae* grows on destroyed plant tissues, damaged mechanically or by small wounds created by pests feeding on plants. The mechanisms of pathogenicity can be divided into: the ability to invade plants using flagella and pilli, the ability to overcome host resistance, and the ability to form biofilms. *P. syringae* has a number of virulence factors, called effector proteins, which are secreted into plant cells with the type III secretion system. These proteins manipulate the immune response of the host to facilitate infection. The action of *P*. *syringae* can also rely on the release of toxins and enzymes degrading cell walls. The secreted phytotoxins, e.g., coronatine, syringomycin, syringopeptin, tabtoin, act as virulence determinants between the plant and the bacterium (Bender, 1999). The specific strain *Pseudomonas syringae* causes, in particular plants, diseases called bacterioses. *P. syringae* is a pathogen of, i.a., tomato, peas, cucumber, soy, cauliflower, beans and fruit trees (Martin-Sanz, 2013; Río-Álvarezh, 2013).

Another example of a plant pathogen is *Pectobacterium carotovorum,* a bacterium located on the 10th place of the "Molecular Plant Pathology" ranking list. It belongs to the *Enterobacteriaceae* family, (formerly included in the genus *Erwinia*)*.* The species *Pectobacterium carotovorum* is geographically widespread. It causes a disease called bacterial soft rot among many food plants, which causes significant economic losses during their cultivation. It is a pathogen of carrots (hence the name carotovora - "carrot"), potato, tomato, green leafy vegetables, pumpkin, onion and others. It occurs on plant surfaces as well as in soil and can infect plants through damaged areas or the naturally occurring pores in the plant. After a successful invasion of the plant, it is found in the extracellular matrix or in the vascular tissue, where it produces pectinolytic enzymes. The produced enzymes hydrolyze pectin, which is one of the basic polymers that build the cell wall of plants, thus causing degradation of the plant's cell wall (Lee, 2013). The above feature constitutes a determinant of pathogenicity of *Pectobacterium carotovorum.*

S. Jafra et al,JOURNAL OF APPLIED MICROBIOLOGY, vol. 106(1), pages 268-277, 2009 discloses potential of bulb-associated bacteria for biocontrol of hyacinth soft rot caused by *Dickeya zeae.* US 2016374341 discloses use of combinations comprising host defense inducers and biological control agents for controlling bacterial harmful organisms in useful plants. Milva Pepi et al, Extremophiles, vol. 13(5), pages 839-848, 2009 describes two naphthalene degrading bacteria belonging to the genera *Paenibacillus* and *Pseudomonas* isolated from a highly polluted lagoon perform different sensitivities to the organic and heavy metal contaminants.

In order to control losses caused by pathogens, chemical plant protection agents are used. The use of such agents, which are collectively referred to as pesticides, began at the end of the 19th century. Bactericides are used as the phytopathogen control agents. Unfortunately, their activity is not limited only to the control of bacteria pathogenic to the plant, but it also affects the number and diversity of soil microorganisms, which are an important component of the soil environment and carry out very important functions in the soil. Microorganisms, and especially bacteria, play a key role in the decomposition of organic matter, during the cycling of elements in the natural environment and the maintenance of soil fertility. Microorganisms improve the general condition of plants. There are populations of bacteria that live around the roots of the plants supplying them with biogenic substances. Bacteria that live in the soil also protect the plant against pathogens (Jastrzebska, 2010). The change in the activity of soil microorganisms caused by the use of pesticides ultimately leads to disturbances in the soil ecosystem and loss of soil fertility. Numerous studies have been carried out which underline these adverse effects of pesticides on soil bacteria and soil respiration (Lo, 2010). The most unfavorable are pesticides: bactericides - antibacterial agents, fungicides - antifungal agents and herbicides - weed killers. Estimates provided by the CIECH Group - the leader of the Polish chemical market - indicate that the amount of plant protection products used in Poland varies in the range of 70-90 thousand tons per year.

The soil layer directly surrounding the root system is referred to as rhizosphere and the term "rhizobacteria" means a group of bacteria capable of colonizing the root environment (Ahemad, 2014). Microbiological cooperation in the rhizosphere is of key importance for the durability and growth of plants. Plants produce a wide range of organic compounds, including sugars, organic acids and vitamins that can be used as nutrients or signals by microbial populations. On the other hand, bacteria in the soil make the nutrients available to the plants and provide additional benefits, such as more effective minerals and water uptake, resistance to diseases, protection against heavy metals toxicity and improved soil structure. Plant Growth Promoting Microorganisms (PGPM) are defined as bacteria and fungi that stimulate plant growth and plant protection (Sripontan, 2014). The most widespread PGPM group are Plant Growth Promoting Rhizobacteria (PGPR), colonizing the soil root zone or as endophytes living in the surface tissues of the roots (Compant, 2005). These microorganisms affect the development of plants in a direct or indirect way. Direct mechanisms include providing nutrients to the plant, synthesis of biologically active substances - plant growth regulators and lowering the level of ethylene, which adversely affects the rooting of plants. Bacteria can also act indirectly by eliminating phytopathogens that adversely affect plant growth or by eliminating compounds that inhibit plant growth. PGPR control plant pathogens by competing for an ecological niche and minerals. They can also produce antibiotics, metabolites/bacteriocins or enzymes that lyse the cell wall of phytopathogens (Glick, 2012; Vejan, 2016).

A broad range of bacteria living in the rhizosphere can produce plant hormones such as auxins, cytokinins and gibberellins, which are substances regulating the growth and development of plants. The effect of auxins is dependent on their concentration and is based i.a., on: stimulation of division and elongation of plant cells, extension of the primary root, stimulation of lateral and adventitious root formation. The consequence of this is the increased surface area as well as the length of the roots, thanks to which plants have better access to nutrients in the soil (Glick, 2012). Bacteria are able to improve the assimilation of nitrogen, phosphorus and iron by plants, and as a result stimulate the growth of plants.

Microorganisms are one of the most important factors determining the fertility and biological productivity of soils. The key roles of bacteria in the soil include:
- cycling of elements in the environment,
- decomposition of organic matter, including many toxic compounds,
- formation of humus (it is necessary for the proper growth of plants, retention of water in the soil),
- binding of atmospheric nitrogen (in 'healthy' soils, bacteria are able to bind even 500 kg of nitrogen from 1 ha) and its conversions (ammonification, denitrification, nitrification),
- increasing the availability of bioelements,
- biostimulation of the root system by secretion of phytohormones,
- inhibition of the development of plant pathogens (bacteria pathogenic to plants, fungi of the genus *Fusarium*) through the secretion of metabolites/bacteriocins.

Due to the negative effects of the use of pesticides, the current trend on a global scale seems to be the reduction of the use of chemical plant protection products by searching for safe and ecological alternatives. Soil bacteria that produce natural compounds - bacteriocins seem to be a very good alternative to chemical plant protection products. Strains of bacteria producing bacteriocins can be effectively used in agriculture, reducing the amount of mineral fertilizers and chemicals, such as antibacterial or fungicidal agents, used (Subramanian, 2015).

### Bacteriocins

Bacteriocins are peptides or small-molecule proteins that can act in a bactericidal or bacteriostatic way. They are produced by various bacterial species, both Gram-positive and Gram-negative, as well as archaea (Riley 2002). Microorganisms that produce bacteriocins also have resistance to them. It results from the construction of bacteriocin operons, which are equipped with genes coding for an active protein that ensures the producer's insensitivity to the compounds produced. The bacteriocin operon also contains genes responsible for transporting bacteriocin from the cell and sometimes genes encoding enzymes for post-translational modification of bacteriocins.

Bacteriocins have a small molecular weight, rarely more than 10 kDa (Zacharow, 2012). They are produced by bacteria to control other microorganisms that may belong to the same or related species as the producer (Cotter, 2005). It follows that they have a narrow scope of action, however, some of them show a significantly extended spectrum, including pathogenic organisms. As peptides, they are generally resistant to chemical and thermal degradation. Bacteriocins are a very diverse group of polypeptides. The differences between them concern biochemical properties, thermal stability, molecular mass, mechanisms of action, extracellular secretion mechanisms, or even the way of protecting the producer against the destructive action of the bacteriocin (Hammami, 2013). Bacteriocins are usually produced under stress conditions, which results in the rapid elimination of neighboring cells that are not immune to their action (Eunjung, 2010). There are studies that indicate that bacteriocins contribute to increased competition dynamics between different strains of bacteria, which significantly affect the maintenance of microbial diversity (Riley, 2002).

### DESCRIPTION OF THE INVENTION

In the light of the described state of the art, the aim of the present invention is to overcome the indicated inconveniences and to provide a composition comprising isolated strains of saprophytic soil bacteria: *Arthrobacter* sp. strain 1.4, *Bacillus* sp. strain 202B, *Bacillus* sp. strain 233, *Bacillus* sp. strain 307A, *Citrobacter* sp. strain 6A, *Peanibacillus* sp. strain 45, *Pseudomonas* sp. strain 23 (W), *Pseudomonas* sp. strain 60 (W), which can be used to fertilize the soil and to control plant pathogens in the soil and restore the natural biological balance of soil microflora, especially after intensive use of chemical plant protection products, pesticides, that biostimulates plant development and growth and the method of using thereof. The object of the invention is therefore the composition comprising isolated strains of saprophytic soil bacteria: *Arthrobacter* sp. strain 1.4, *Bacillus* sp. strain 202B, *Bacillus* sp. strain 233, *Bacillus* sp. strain 307A, *Citrobacter* sp. strain 6A, *Peanibacillus* sp. strain 45, *Pseudomonas* sp. strain 23 (W), *Pseudomonas* sp. strain 60 (W), deposited on December 19, 2017 under the deposit number B/00148 in the Polish Collection of Microorganisms PCM, Institute of Immunology and Experimental Therapy PAS, Wroclaw, Poland.

The invention also relates to the biopreparation comprising the composition according to the invention. The biopreparation preferably additionally contains liquid mineral medium supplemented with glucose as the sole carbon source or solid mineral medium supplemented with glucose as the sole carbon source.

The biopreparation preferably contains at least 10⁵ bacterial cells per ml of the medium, more preferably at least 10⁶ bacterial cells per ml of the medium, more preferably the cfu of each strain is at least 10⁶ per ml of the medium.

In the biopreparation, the strains *Arthrobacter* sp. strain 1.4, *Bacillus* sp. strain 202B, *Bacillus* sp. strain 233, *Bacillus* sp. strain 307A, *Citrobacter* sp. strain 6A, *Peanibacillus* sp. strain 45, *Pseudomonas* sp. strain 23 (W), *Pseudomonas* sp. strain 60 (W) are in a substantially equal quantitative ratio to each other.

The biopreparation is in the form of liquid or lyophilisate, or embedded in a carrier. The invention also relates to the method of production of the composition comprising isolated strains of saprophytic soil bacteria according to the invention and/or biopreparation according to the invention, in which the culturing of strains *Arthrobacter* sp. strain 1.4, *Bacillus* sp. strain 202B, *Bacillus* sp. strain 233, *Bacillus* sp. strain 307A, *Citrobacter* sp. strain 6A, *Peanibacillus* sp. strain 45, *Pseudomonas* sp. strain 23 (W), *Pseudomonas* sp. strain 60 (W) deposited as B/00148, is carried out
a) simultaneously in a medium providing the growth of each of the strains, or
b) each of the strains is cultured individually in an appropriate medium enabling its growth, and the obtained cultures are mixed in order to obtain the composition comprising the isolated strains of saprophytic soil bacteria and/or the biopreparation, preferably each strain is mixed with the other in a substantially equal quantitative ratio,
   wherein, preferably, the culture in a) and/or b) is carried out at a temperature in the range of 20-25°C.

In the preferred method of production of the composition and/or the biopreparation, the medium used at the step of culturing a) and/or b) is liquid mineral medium supplemented with glucose as the sole carbon source or solid mineral medium supplemented with glucose as the sole carbon source.

The invention also relates to the method of plant pathogen control in soil comprising the step of introducing of the composition comprising the isolated strains of saprophytic soil bacteria according to the invention and/or the biopreparation according to the invention and/or the composition and/or the biopreparation produced by the method of production thereof according to the invention into the ground, wherein, preferably, the introduction into the ground is carried out by sprinkling (spraying).

The method of plant pathogen control in soil is preferred when the pathogen is selected from *Pseudomonas syringae, Pectobacterium carotovorum,* wherein
more preferably *Pseudomonas syringae* is a pathogen of tomato, peas, cucumber, soy, cauliflower, beans and fruit trees, potato, carrot, pepper, and/or
more preferably *Pectobacterium carotovorum* is a pathogen of carrot, potato, tomato, pepper, green leafy vegetables, pumpkin, onion.

Preferably, the method of plant pathogen control in soil takes place *ex situ* or takes place *in situ.*

In the preferred method of plant pathogen control in soil, the soil is sprinkled with the biopreparation in the form of diluted preparation in the amount of 10 liters per 1 ha of land surface and/or the biopreparation is introduced into the ground in a volume ratio biopreparation: soil in the range from 1:10 to 1:30, wherein the initial preparation used for sprinkling and/or introducing into the ground is preferably diluted in a ratio 1:50 to 1:100.

Equally preferably, the method of plant pathogen control in soil additionally comprises the steps - mechanical oxygenation of soil and humidification of soil to maintain the appropriate soil moisture level.

The invention also relates to the method of soil fertilization and restoring the natural biological balance of soil microflora comprising the step of introducing of the composition comprising the isolated strains of saprophytic soil bacteria according to the invention and/or the biopreparation according to the invention and/or produced by the method of production according to the invention into the ground, wherein, preferably the introduction into the ground is carried out by sprinkling, preferably after intensive use of chemical plant protection products.

Preferably, the method of soil fertilization and restoring the natural biological balance of soil microflora takes place *ex situ* or takes place *in situ.*

In the preferred method of soil fertilization and restoring the natural biological balance of soil microflora, the biopreparation in the form of diluted preparation is introduced into the ground in the amount of 10 liters per 1 ha of land surface and/or in a volume ratio biopreparation: soil in the range from 1:10 to 1:30, wherein the initial preparation used for sprinkling and/or introducing into the ground is preferably diluted in a ratio 1:50 to 1:100.

The invention also relates to the method of biostimulation of plant development and growth comprising the step of introducing of the composition comprising the isolated strains of saprophytic soil bacteria according to the invention and/or the biopreparation according to the invention and/or produced by the method of production according to the invention into the ground, wherein, preferably the introduction into the ground is carried out by sprinkling.

Preferably, the method of biostimulation of plant development and growth takes place *ex situ* or takes place *in situ.*

In the preferred method of biostimulation of plant development and growth, the biopreparation in the form of diluted preparation is introduced into the ground in the amount of 10 liters per 1 ha of land surface and/or in a volume ratio biopreparation: soil in the range from 1:10 to 1:30, wherein the initial preparation used for sprinkling and/or introducing into the ground is preferably diluted in a ratio 1:50 to 1:100.

The method of biostimulation of plant development and growth is preferably carried out for plants selected from tomato, peas, cucumber, soy, cauliflower, beans and fruit trees, carrot, potato, tomato, green leafy vegetables, pumpkin, onion, pepper.

The invention also relates to use of the composition comprising the isolated strains of saprophytic soil bacteria according to the invention and/or the biopreparation according to the invention and/or produced by the method of production according to the invention for plant pathogen control in soil.

In the preferred embodiment of use in plant pathogen control in soil, the pathogen is selected from *Pseudomonas syringae, Pectobacterium carotovorum,*
more preferably *Pseudomonas syringae* is a pathogen of tomato, peas, cucumber, soy, cauliflower, beans and fruit trees, potato, carrot, pepper, and/or
more preferably *Pectobacterium carotovorum* is a pathogen of carrot, potato, tomato, pepper, green leafy vegetables, pumpkin, onion.

The invention also relates to use of the composition comprising the isolated strains of saprophytic soil bacteria according to the invention and/or the biopreparation according to the invention and/or produced by the method of production according to the invention for soil fertilization and restoring the natural biological balance of soil microflora, preferably after intensive use of chemical plant protection products.

The invention also relates to use of the composition comprising the isolated strains of saprophytic soil bacteria according to the invention and/or the biopreparation according to the invention and/or produced by the method of production according to the invention for biostimulation of plant development and growth, wherein in such use plants are preferably selected from tomato, peas, cucumber, soy, cauliflower, beans and fruit trees, carrot, potato, pepper, green leafy vegetables, pumpkin, onion.

The present solution constitutes a natural method of controlling phytopathogens: *Pseudomonas syringae* and *Pectobacterium carotovorum* present in the soil by bactericidal action. Bacteriocins, responsible for the bactericidal activity, are secreted by the strains included in the biopreparation: *Bacillus* sp. strain 202B, *Bacillus* sp. strain 233, *Bacillus* sp. strain 307A, *Citrobacter* sp. strain 6A, *Peanibacillus* sp. strain 45, *Pseudomonas* sp. strain 23 (W), *Pseudomonas* sp. strain 60 (W). The same strains are capable of degrading organic matter to compounds easily assimilable by plants, constituting sources of, e.g., C, N and P, and are insensitive to heavy metals except for cadmium (Cd). *Arthrobacter* sp. strain 1.4 is capable of binding atmospheric nitrogen and thus enriching the soil with nitrogen; reduction of the toxic hexavalent chromium Cr(VI) and is insensitive to other heavy metals - nickel Ni(II) and copper Cu(II), as well as pesticide degrading. The strains of the genera *Bacillus* and *Pseudomonas* included in the composition deposited as B/00148 have properties that are their generic features: amylolytic (decomposition of starch), proteolytic (protein degradation), denitrifying (reduction of nitrates to nitrogen) and ammonifying (conversion of nitrogen contained in organic compounds). The composition deposited as B/00148 as well as the biopreparation produced on its basis according to the invention simultaneously fertilizes the soil and restores the natural biological balance of soil microflora, especially after intensive use of chemical plant protection products such as pesticides. Through comprehensive action, the composition deposited as B/00148 and the biopreparation based on it biostimulates plant development and growth.

The selected strains of bacteria included in the composition deposited as B/00148 were isolated from soils, they are not pathogenic and they do not comprise genetic modifications. The processes that are carried out by these strains occur naturally in the soil environment. The developed, selected composition of the composition and biopreparation provides the possibility to quickly propagate the appropriate amount of the preparation. The effect of bactericidal activity is visible after a period of just 14 days from the administration of the composition and/or biopreparation to the soil. The recommended dose and dosing intervals depends on the type and condition of the soil. It is obvious that the more damaged is the soil, as a result of the use of chemical plant protection products, the more preparation it is worth to apply. For example, the ranges of preparation used for slightly damaged soils can vary between 0.2-2 L/ha, preferably about 1 L/ha, and for damaged soils from about 5 to about 20 L/ha, preferably about 10 L/ha.

The object of the invention is also the method of using the biopreparation in soil, based on that the selected soil microorganisms in the form of biopreparation composition according to the invention are introduced into the ground, and the soil is mechanically oxygenated and the appropriate level of its humidity is maintained, thereby eliminating the bacteria pathogenic to plants: *Pseudomonas syringae* and *Pectobacterium carotovorum* from soil.

The use of the composition and biopreparation being the object of the invention is a completely natural method, especially recommended for use in organic farming, which does not introduce any synthetic products into the environment. The fact that the biopreparation composition according to the invention consists of selected and appropriately arranged strains, mutually not disturbing their own development and the growth of autochthonous organisms, shortens the time needed for propagation and metabolic activity of bacteria, moreover the method is based on processes that occur naturally in the environment, which are effective and more economical than, for example, chemical methods.

### BRIEF DESCRIPTION OF DRAWINGS

For a better understanding of the invention, it has been illustrated in the embodiments and in the accompanying figures, in which:

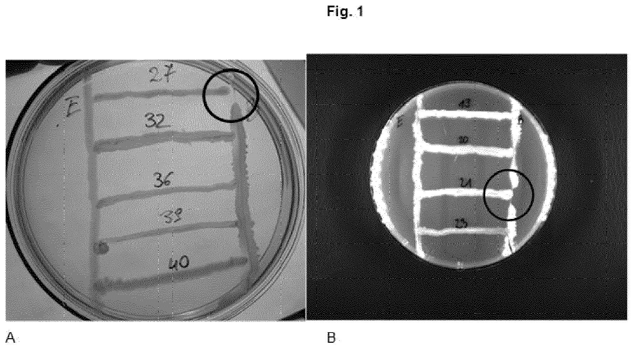

**Fig.1** Shows an exemplary visualization of phytopathogen growth inhibition zones in a ladder test (A and B).

### EXAMPLES

The following examples are presented merely to illustrate the invention and to clarify its various aspects, but are not intended to be limitative, and should not be equated with all its scope, which is defined in the appended claims. In the following examples, unless it was otherwise indicated, standard materials and methods used in the art were used, or the manufacturers' instructions for specific materials and methods were followed.

### Example 1. Selection of the biopreparation composition

563 strains of soil bacteria were isolated from different arable soils from organic farms as well as those using chemical plant protection chemicals from the Mazovian Voivodeship. Their identification to the genus/species was carried out using PCR amplification of the 16S rRNA gene, sequencing and bioinformatic analyzes. From the available pool of strains, bacteria which are not pathogens of humans, animals and plants were selected for further studies. The further stages of the studies were based on the determination of the metabolic properties of the selected bacterial strains.

The overriding goal was to find soil bacteria capable of effective bacteriostatic / bactericidal action against two plant pathogens: *Pseudomonas syringae* and *Pectobacterium carotovorum.*

Intermediate goals were: the selection of bacteria with various valuable metabolic properties for the functioning of biodiversity and proper soil microflora (capability to bind nitrogen, carry out the processes of denitrification, nitrification and ammonification, having proteolytic and cellulolytic properties - the ability to break down animal and plant debris), as well as for proper development and increase in agricultural yield (provision of easily assimilable mineral compounds for plants, bioelements: nitrogen, carbon, potassium and phosphorus). The strains have been grouped into specific compositions to show a synergistic effect. From the tested, the most effective composition of the selected bacterial strains is the object of this application.

Due to their complementary properties and synergistic interaction, the following isolated microorganisms included in the biopreparation composition were selected **(Table 1).**

**Table 1. Labelling of the selected strains included in the composition/biopreparation deposited as B/00148**

| **No.** | **Strain label** | **Bacteria genus** |
|---|---|---|
| 1 | 1.4 | *Arthrobacter* sp. |
| 2 | 202B | *Bacillus* sp. |
| 3 | 233 | *Bacillus* sp. |
| 4 | 307A | *Bacillus* sp. |
| 5 | 6A | *Citrobacter* sp. |
| 6 | 45 | *Peanibacillus* sp. |
| 7 | 23 (W) | *Pseudomonas* sp. |
| 8 | 60 (W) | *Pseudomonas* sp. |

### Example 2. Determination of the mutual antagonistic effect of the strains constituting the biopreparation composition deposited as B/00148

To determine whether the individual strains do not exert any antagonistic effect on each other (inhibition of the growth of one strain by the other), the bacteria were plated by single line streaking using a sterile loop on Petri dishes with Davis medium prepared from the following ingredients: 150 ml of distilled water or 150 ml of agaroid (distilled water + agar) (depending on the consistency of the medium); 40 ml of Davis salts; 4 ml of 20% glucose. The specific bacterial strains were plated at a distance of about 2 mm from each other in fixed configurations. The developed systems were designed to verify whether each of the strains is capable of growth in the immediate vicinity of other strains. The results were read after 24 h of incubation at 22 °C. Each of the tested strains was able to grow in the immediate vicinity of the others. Based on this it can be concluded that between the microorganisms included in the composition deposited as B/00148 there is no antagonistic effect, i.e. none of the tested strains changes the environment in such a way as to inhibit the growth of others and lead to bacteriostasis.

### Example 3. Determination of the metabolic properties of the strains included in the composition/biopreparation deposited as B/00148 (standard experimental methods were used)

### A. Degradation of organic matter of plant origin

Lignocellulose biomass degradability tests associated with the production of cellulolytic enzymes is carried out using plant debris, e.g., crop residues or fragments of plant biomass used for composting. The positive result of biodegradability of cellulose-derived biomass is the conversion of this biomass to humus as well as the presence of glucose. The formation of humus is assessed by appearance, and the presence of glucose (C₆H₁₂O₆) is determined colorimetrically with glucose oxidase according to a standard method using a diagnostic kit (Lquick Cor-GLUCOSE) after 14 days of incubation at 22°C. It was shown that the strains included in the biopreparation: *Bacillus* sp. strain 202B, *Bacillus* sp. strain 233, *Bacillus* sp. strain 307A, *Peanibacillus* sp. strain 45, *Pseudomonas* sp. strain 23 (W), *Pseudomonas* sp. strain 60 (W) are capable of degrading organic matter to compounds easily assimilable by plants, being the source of, e.g., C, N and P.

### B. Atmospheric nitrogen binding

Atmospheric nitrogen binding is associated with the reduction of nitrogen N₂ to ammonia NH₃. The test is carried out in a liquid medium that does not contain a source of nitrogen. The positive result is a) increase in the density of the culture after 24 h of incubation at 22 °C measured by the means of a spectrophotometer, and b) the characteristic smell of ammonia and alkalization of the medium as measured by a pH indicator. It was shown that the strain *Arthrobacter* sp*.* 1.4 is capable of atmospheric nitrogen binding.

### C. Proteolytic, amylolytic, denitrifying and ammonifying properties

Proteolytic properties were tested using solid media: nutrient agar with 4% non-fat milk. To test the amylolytic properties, solid medium was used: nutrient agar with the addition of 1% starch. Each of the strains of the genera *Bacillus* and *Pseudomonas* was plated by single line streaking. The results were read after 24 h of incubation at 22 °C. The ability to degrade proteins, manifested by the appearance of a clear zone of the medium around the bacterial growth line, has been demonstrated by the strains of the genera *Bacillus* and *Pseudomonas* included in the composition deposited as B/00148. Amylolytic properties, where the positive result was the lack of violet color around the bacterial growth line after pouring Lugol's liquid onto the plates, have been demonstrated by the strains of the genera *Bacillus* and *Pseudomonas* included in the composition, i.e.: *Bacillus* sp. strain 202B, *Bacillus* sp. strain 233, *Bacillus* sp. strain 307A, *Pseudomonas* sp. strain 23 (W), *Pseudomonas* sp. strain 60 (W).

Proteolytic properties were tested using a liquid medium: nutrient broth with 0.1% KNO₃. The medium is poured into long glass test tubes in which a Durham tube is also placed. The denitrification ability of the strain is indicated by the accumulation of gas (nitrogen) in the Durham tube and alkalization of the medium determined by a pH indicator after 24 h of incubation at 22 °C. The ability to carry out the denitrification process (reduction of nitrates to nitrogen) has been demonstrated by strains of the genera *Bacillus* and *Pseudomonas* included in the biopreparation, i.e.: *Bacillus* sp. strain 202B, *Bacillus* sp. strain 233, *Bacillus* sp. strain 307A, *Pseudomonas* sp. strain 23 (W), *Pseudomonas* sp. strain 60 (W).

Ammonifying properties, that is the process of conversion of nitrogen contained in organic compounds of plant or animal origin to ammonia and ammonium ions (NH₄⁺), were tested using a liquid medium: nutrient broth with 4% non-fat milk or urea. The presence of ammonia and ammonium ions is indicated by the characteristic odor and alkalization of the medium. The addition of phenol red to the medium changes the color of the medium from yellow to red, which indicates that the pH is alkaline. Ammonifying properties have been demonstrated by the strains of the genera *Bacillus* and *Pseudomonas* included in the preparation composition, i.e.: *Bacillus* sp. strain 202B, *Bacillus* sp. strain 233, *Bacillus* sp. strain 307A, *Pseudomonas* sp. strain 23 (W), *Pseudomonas* sp. strain 60 (W).

### Example 4. Determination of the sensitivity to heavy metals of the strains constituting the composition

In the studies, the concentrations of heavy metals in the range of 0.2-30 mM were used. For this purpose, immediately before use, the liquid medium (nutrient broth) was supplemented with an appropriate volume of the concentrated stock solution to obtain the final concentration given in **Table 2** for particular metals. NaAsO₂ for As(III), NiCl₂x6H₂O for Ni(II), Cr₂K₂O₇ for Cr(VI), ZnSO₄x7H₂O for Zn(II), CdSO₄x8H₂O for Cd(II), CuSO₄ for Cu(II) were used.

**Table 2. The range of concentrations of metal salt solutions in nutrient broth (NB)**

| As(III), Cu(II), Cr(VI), Ni, Zn | 2 mM | 4 mM | 8 mM | 6 mM | 8 mM | 10 mM | 12 mM | 14 mM | 16 mM | 18 mM | 20 mM | 30 mM |
|---|---|---|---|---|---|---|---|---|---|---|---|---|
| Cd | 0.2 mM | 0.4 mM | 0.8 mM | 0.6 mM | 0.8 mM | 1.0 mM | 1.2 mM | 1.4 mM | 1.6 mM | 1.8 mM | 2.0 mM | 3.0 mM |

The sensitivity of the isolated strains to heavy metals was tested by determining the MIC values. For this purpose, 96-well titration plates (ROTH) were used. 150 µl of a metal salt solution in NB was pipetted into each well, so as to form a series with increasing concentration of each metal (gradient), and then they were inoculated with 150 µl of inoculum obtained from cultures of the individual strains with a density of 0.5 McFarland. In each of the 8 rows (12 wells) there was a different concentration of a given metal, according to the **Table 2,** wherein the last row of microtubes constituted a negative control (NB medium with saline solution, or NB with metal stock solution without the inoculum).

The plates were covered with a plastic cap and additionally wrapped in cellophane foil to protect them from evaporation. The whole was incubated in a shaker (80 rpm) at the temperature of 22 °C. After 24 and 48 h of incubation, the optical density of the culture (OD₆₀₀) was measured in each of the 96 wells. The "Sunrise" (TECAN) spectrophotometer was used to carry out the measurements.

The values of the minimum metal concentration inhibiting bacterial growth (MIC) were determined.

The MIC value was assumed as the lowest concentration at which no bacterial growth was observed. The results are presented in **Table 3.**

**Table 3. MIC values for heavy metals for the investigated strains**

| No. | Strain label | MIC limit value [mM/L], 24h(48h) | | | | | |
|---|---|---|---|---|---|---|---|
| | | Cu(II) | Ni(II) | Zn(II) | Cd(II) | As(III) | Cr(VI) |
| 1 | 1.4 | 2.5 | 2.5 | 2 | 1 | 0.6 | 0.5 |
| 2 | 202B | 5 | 3 | 1.5 | 0.5 | 4 | 0.5 |
| 3 | 233 | 5 | 3 | 1.5 | 0.5 | 4 | 0.5 |
| 4 | 307A | 4 | 3 | 1.5 | 0.5 | 3(4) | 0.5 |
| 5 | 6A | 5 | 3 | 1.5 | 0.5 | 4(5) | 0.5 |
| 6 | 45 | 5 | 3 | 1.5 | 0.5 | 4(5) | 0.5 |
| 7 | 23 (W) | 5 | 3 | 1.5 | 0.5 | 4 | 0.5 |
| 8 | 60 (W) | 5 | 3 | 1.5 | 0.5 | 3(4) | 0.5 |

**Table 4. MIC values determined for the reference strain Escherichia coli, according to Spain, 2003**

| | | | |
|---|---|---|---|
| Solutions metal salts in (NB): | As(III), Cr(VI), Ni(II), Zn(II), Cu(II): | Heavy metal | MIC [mM/L] |
| | 1 mM, 2 mM, 3 mM, 4 mM, 5 mM, 6 mM, | | |
| | 7 mM, 8 mM, 9 mM, 10 mM, 15 mM | As(III) | 0.5 |
| | | Cu(II) | 1 |
| | Cd(II): | Ni(II) | 1 |
| | | 'Zn(II) | 1 |
| | 0.1 mM, 0.2 mM, 0.3 mM, 0.4 mM, 0.5 mM, | Cd(II) | 0.5 |
| | 0.7 mM, 0.8 mM, 0.9 mM, 1.0 mM, 1.5 mM | Cr(VI) | 0.2 |

The obtained results indicate that the tested bacterial strains are resistant to Ni(II), Cu(II), Zn(II), As(III), semi-sensitive to Cr(VI) and sensitive to Cd(II). It should be emphasized that in the case of arsenic and chromium, the metal form considered to be the most toxic to living organisms (As(III) and Cr(VI)) was used, and yet high MIC values were observed for the tested strains compared to literature data for the reference strain **(Table 4)** (Spain, 2003).

### Example 5. Bactericidal activity against phytopathogens: plate test - ladder test

The strains included in the biopreparation deposited as B/00148, in the form of pure cultures, have been tested for the ability to inhibit the growth of strains of phytopathogenic bacteria: *Pseudomonas syringae* and *Pectobacterium carotovorum.* For each strain, ladder streaking was performed to observe their bacteriostatic/bactericidal activity **(****Fig.1****).** After a 24-hour incubation at the temperature of 22 °C, the first reading, which consisted of a visual evaluation of the inhibition zones, was performed. The investigated strains potentially producing metabolites/bacteriocins inhibited the growth of phytopathogens. The degree of inhibition was assessed using a three-point scale (+, ++, +++). The inhibition zones were observed and read after 48 h and 72 h. The obtained results are presented in **Table 5.**

**Table 5. Analysis of the phytopathogens growth inhibition zones by the tested strains**

| Strain number | Strain genus | *Pseudomonas syringae* | *Pectobacterium carotovorum* |
|---|---|---|---|
| 1.4 | *Arthrobacter sp.* | - | - |
| 202B | *Bacillus sp.* | +++ | +++ |
| 233 | *Bacillus sp.* | +++ | ++ |
| 307A | *Bacillus sp.* | - | ++ |
| 6A | *Citrobacter sp.* | - | ++ |
| 45 | *Peanibacillus sp.* | + | + |
| 23 (W) | *Pseudomonas sp.* | +++ | - |
| 60 (W) | *Pseudomonas sp.* | ++ | - |
| The inhibition zones were defined using the following scale: + = 0.1-1 mm, ++ = 1.1-2.1 mm, 2.2-3 mm, +++ = complete growth inhibition, - = lack of growth inhibition | | | |

Bactericidal activity was also confirmed using the method of testing the activity of culture supernatants of bacteria producing bacteriocins against phytopathogens - a plate test. *Pseudomonas syringae* and *Pectobacterium carotovorum* with a density of 0.5 McFarland were plated in such a way that after 24 h of incubation at the temperature of 22 °C, the bacteria would grow in the form of a homogeneous lawn. Before incubation, culture supernatants of all the eight test bacterial strains (separately) were administered onto symmetrically arranged sterile paper discs placed on the prepared plates. In the case of seven of the tested strains, a zone of inhibition of growth of phytopathogenic bacteria was observed. Three of the tested strains inhibit the growth of both phytopathogens, two inhibit only *Pectobacterium carotovorum* and the other two strains are active only against *Pseudomonas syringae.* The results are shown in **Table 6,** and they are consistent with those obtained in the ladder test.

**Table 6. Analysis of the phytopathogens growth inhibition zones by the tested strains**

| Strain number | Strain genus | *Pseudomonas syringae* | *Pectobacterium carotovorum* |
|---|---|---|---|
| 1.4 | *Arthrobacter sp.* | - | - |
| 202B | *Bacillus sp.* | + | + |
| 233 | *Bacillus sp.* | + | + |
| 307A | *Bacillus sp.* | - | + |
| 6A | *Citrobacter sp.* | - | + |
| 45 | *Peanibacillus sp.* | + | + |
| 23 (W) | *Pseudomonas sp.* | + | - |
| 60 (W) | *Pseudomonas sp.* | + | - |
| The inhibition zones were defined using the following scale: + = growth inhibition, - = lack of growth inhibition | | | |

### Example 6. Bactericidal activity against phytopathogens - pot tests for various plant species

The pot experiment was carried out using the strains included in the biopreparation following their propagation (overnight culture) in two versions: for each strain as well as a mixture of all the strains, after mixing them in the same volume ratio and taking into account the bacterial count (suspensions of all the strains were characterized by similar optical density measured spectrophotometrically). The experiment was carried out to assess the activity of individual strains as well as the biopreparation in the natural soil environment. The experiment was carried out in two variants; A. for *Pectobacterium carotovorum* and B. for *Pseudomonas syringae.* Each pot with garden soil was inoculated with 30 ml of the overnight culture of *Pectobacterium carotovorum* or *Pseudomonas syringae,* after 24 hours 30 ml of the biopreparation composition deposited as B/00148 or 30 ml, separately, of each strain included in the composition was added. Additionally, a positive control - the soil without the addition of any bacteria (30 ml of saline solution was added); a negative control - the soil inoculated with *Pectobacterium carotovorum* or *Pseudomonas syringae* (30 ml of overnight culture) and a sample where only single strains or the mixture of strains (biopreparation deposited as B/00148) (30 ml) were added without pre-inoculation with the phytopathogens were prepared. In total, 10 control samples (controls), 2 variants (A and B) for the mixture of strains and 16 variants (A and B) for individual strains were obtained **(Table 7),** each of which was repeated at least three times.

**Table 7. Pot experiment variants.**

| **Controls K** | **Variant A** | **Variant B** |
|---|---|---|
| 1K. positive control | 1A. negative control- soil inoculated with *Pectobacterium carotovorum* | 1B. negative control- soil inoculated with *Pseudomonas syringae* |
| 2K. Sample where only the biopreparation (composition B/00148) was added | 2A. soil inoculated with *Pectobacterium carotovorum* + the biopreparation (composition B/00148) added after 24 h | 2B. soil inoculated with *Pseudomonas syringae* + the biopreparation (composition B/00148) added after 24 h |
| 3K. Sample where only the strain 1.4 was added | 3A. soil inoculated with *Pectobacterium carotovorum* + strain 1.4 added after 24 h | 3B. soil inoculated with *Pseudomonas syringae* + strain 1.4 added after 24 h |
| 4K. Sample where only the strain 202B was added | 4A. soil inoculated with *Pectobacterium carotovorum* + strain 202B added after 24 h | 4B. soil inoculated with *Pseudomonas syringae* + strain 202B added after 24 h |
| 5K. Sample where only the strain 233 was added | 5A. soil inoculated with *Pectobacterium carotovorum* + strain 233 added after 24 h | 5B. soil inoculated with *Pseudomonas syringae* + strain 233 added after 24 h |
| 6K. Sample where only the strain 307A was added | 6A. soil inoculated with *Pectobacterium carotovorum* + strain 307A added after 24 h | 6B. soil inoculated with *Pseudomonas syringae* + strain 307A added after 24 h |
| 7K. Sample where only the strain 6A was added | 7A. soil inoculated with *Pectobacterium carotovorum* + strain 6A added after 24 h | 7B. soil inoculated with *Pseudomonas syringae* + strain 6A added after 24 h |
| 8K. Sample where only the strain 45 was added | 8A. soil inoculated with *Pectobacterium carotovorum* + strain 45 added after 24 h | 8B. soil inoculated with *Pseudomonas syringae* + strain 45 added after 24 h |
| 9K. Sample where only the strain 23 (W) was added | 9A. soil inoculated with *Pectobacterium carotovorum* + strain 23 (W) added after 24 h | 9B. soil inoculated with *Pseudomonas syringae* + strain 23 (W) added after 24 h |
| 10K. Sample where only the strain 60 (W) was added | 10A. soil inoculated with *Pectobacterium carotovorum* + strain 60 (W) added after 24 h | 10B. soil inoculated with *Pseudomonas syringae* + strain 60 (W) added after 24 h |

Two weeks after the introduction of the appropriate suspensions into the soil, potatoes (tubers), carrot (seeds), tomato, pepper (seedlings) were planted in them, respectively. The plants were grown at the appropriate temperature, with care for proper hydration of the plants.

Potato - the results were collected after 6 weeks: the growth of potato shoots was observed in the case of Control for all samples (1-10), as well as in the case of Variant A for samples: 2, 4, 5 and 6, and in the case of Variant B for samples: 2, 4, 5 and 6. In the variants with the addition of only phytopathogens, no growth (variant B1 soil with *Pseudomonas syringae*) or growth at the minimum level, (variant A1 soil with *Pectobacterium carotovorum*) where the shoot length was only 3 cm, was observed. In addition to the visual assessment, a quantitative assessment was carried out by weighing the shoots from each of the pots **(Table 8).** Only in the variants A2 and B2, the growth of the shoots at a level comparable to the control sample 1 was observed, which indicates the elimination of pathogenic bacteria as a result of the addition of the biopreparation. In the remaining samples (A 3-10 and B 3-10), where single strains of bacteria were added, the growth was at a much lower level, as indicated by the biomass distribution in the range 0.75 - 15.01 and in no case a result at the same level as in the variants A2 and B2 was obtained.

In addition, a slight biostimulation of potato shoots growth was observed in the Controls, samples 3-10 (the difference compared to the control was on average 8%) and a significant one in the sample 2 - the sample where a mixture of all strains included in the biopreparation was added, the difference compared to the control was on average 38%, which indicates the synergistic effect of the bacterial strains included in the biopreparation. In the Variants A, 1 and B, 1 samples where no growth of potato shoots was observed or it was slight, a tuber condition test was carried out. In the case of the variant B, 1 (soil with *Pectobacterium carotovorum*) the tuber was rotten in 70%, and in the case of variant A, 1 (soil with *Pseudomonas syringae*) the tuber was completely decomposed. In each case, the characteristic odor of a given bacterium and the decomposition process was observed.

**Table 8. Quantitative assessment of the growth of potato in individual pots. Experiment variants as in the Table 7.**

| Controls | Biomass (g) | Variant A | Biomass (g) | Variant B | Biomass (g) |
|---|---|---|---|---|---|
| 1 K_potato | 19.56 | 1A_potato | 0.72 | 1 B_potato | 0 |
| 2K_potato | 26.83 | 2A_potato | 22.21 | 2B_potato | 19.74 |
| 3K_potato | 21.15 | 3A_potato | 0.89 | 3B_potato | 2.8 |
| 4K_potato | 21.34 | 4A_potato | 14.08 | 4B_potato | 13.89 |
| 5K_potato | 21.22 | 5A_potato | 14.46 | 5B_potato | 14.21 |
| 6K_potato | 21.36 | 6A_potato | 15.01 | 6B_potato | 1.2 |
| 7K_potato | 20.67 | 7A_potato | 0.75 | 7B_potato | 2.8 |
| 8K_potato | 21.18 | 8A_potato | 13.56 | 8B_potato | 13.02 |
| 9K_potato | 20.23 | 9A_potato | 1.8 | 9B_potato | 11.7 |
| 10K_potato | 20.45 | 10A_potato | 2.1 | 10B_potato | 10.89 |

Carrot - the results were collected 8 weeks after germination: the growth of carrot shoots was observed in the case of Control for all samples (1-10), as well as in the case of Variant A for samples: 2, 4, 5 and 6 and in the case of variant B for samples: 2, 4, 5 and 6. In the variants with the addition of only phytopathogens, no growth was observed (variants A1 and B1, soil with *Pectobacterium carotovorum,* soil with *Pseudomonas syringae,* respectively). In addition to the visual assessment, a quantitative assessment was carried out by weighing the shoots from each of the pots **(Table 9).** Only in the variants A2 and B2, the growth of the shoots at a level comparable to the control sample 1 was observed, which indicates the elimination of pathogenic bacteria as a result of the addition of the biopreparation. In the remaining samples (A 3-10 and B 3-10), where single strains of bacteria were added, the growth was at a much lower level, as indicated by the biomass distribution in the range 1.24 - 13.08 and in no case a result at the same level as in the variants A2 and B2 was obtained. In addition, a slight biostimulation of carrot shoots growth was observed in the Controls, samples 3-10 (the difference compared to the control - 1 was on average 4%) and a significant one in the sample 2 - the sample where a mixture of all strains included in the biopreparation was added, the difference compared to the control was 27%. The obtained results indicate the synergistic effect of strains of bacteria that are included in the biopreparation.

**Table 9. Quantitative assessment of the growth of carrot in individual pots. Experiment variants as in the Table 7.**

| Controls | Biomass (g) | Variant A | Biomass (g) | Variant B | Biomass (g) |
|---|---|---|---|---|---|
| 1 K_carrot | 15.34 | 1A_carrot | 0.72 | 1 B_carrot | 0 |
| 2K_carrot | 19.52 | 2A_carrot | 17.46 | 2B_carrot | 16.82 |
| 3K_carrot | 16.78 | 3A_carrot | 1.34 | 3B_carrot | 2.34 |
| 4K_carrot | 16.36 | 4A_carrot | 12.14 | 4B_carrot | 8.23 |
| 5K_carrot | 15.82 | 5A_carrot | 12.76 | 5B_carrot | 9.26 |
| 6K carrot | 15.21 | 6A carrot | 13.08 | 6B_carrot | 2.28 |
| 7K_carrot | 14.34 | 7A_carrot | 1.25 | 7B_carrot | 2.12 |
| 8K_carrot | 15.32 | 8A_carrot | 11.87 | 8B_carrot | 11.02 |
| 9K_carrot | 16.33 | 9A_carrot | 1.24 | 9B_carrot | 11.30 |
| 10K_carrot | 15.24 | 10A_carrot | 3.18 | 10B_carrot | 10.15 |

Tomato - the results were collected 10 weeks after planting of the seedlings: the growth of tomato shoots was observed in the case of Control for all samples (1-10), as well as in the case of Variant A for samples: 2, 4, 5 and 6 and for variant B for samples: 2, 4, 5 and 6. In the variants with the addition of only phytopathogens, low growth was observed, and changes were noted on the leaves (variants A1 and B1, soil with *Pectobacterium carotovorum,* soil with *Pseudomonas syringae,* respectively). In addition to the visual assessment, a quantitative assessment was carried out by weighing the shoots from each of the pots **(Table 10).** Only in the variants A2 and B2, the growth of the shoots at a level comparable to the control sample 1 was observed, which indicates the elimination of pathogenic bacteria as a result of the addition of the biopreparation. In the remaining samples (A 3-10 and B 3-10), where single strains of bacteria were added, the growth was at a lower level, as indicated by the biomass distribution in the range of 12.39 - 15.54 and in no case a result at the same level as in the variants A2 and B2 was obtained. In addition, a significant biostimulation of tomato shoot growth was observed in the Control, in the sample 2 - the sample where a mixture of all strains included in the biopreparation was added, the difference compared to the control was 20%, in the other controls, samples 3-10, no biostimulation was observed, the growth was at the same level as in the Control - sample 1. The obtained results indicate the synergistic effect of strains of bacteria that are included in the biopreparation.

**Table 10. Quantitative assessment of the growth of tomato in individual pots. Experiment variants as in the Table 7.**

| Controls | Biomass (g) | Variant A | Biomass (g) | Variant B | Biomass (g) |
|---|---|---|---|---|---|
| 1 K_tomato | 22.43 | 1A_tomato | 13.28 | 1 B_tomato | 11.56 |
| 2K_tomato | 26.98 | 2A_tomato | 23.18 | 2B_tomato | 22.47 |
| 3K_tomato | 22.19 | 3A_tomato | 14.45 | 3B_tomato | 13.89 |
| 4K tomato | 22.14 | 4A tomato | 14.76 | 4B tomato | 14.28 |
| 5K_tomato | 22.28 | 5A_tomato | 15.54 | 5B_tomato | 14.27 |
| 6K_tomato | 21.42 | 6A_tomato | 15.21 | 6B_tomato | 14.22 |
| 7K_tomato | 21.89 | 7A_tomato | 11.87 | 7B_tomato | 13.11 |
| 8K_tomato | 22.26 | 8A_tomato | 10.56 | 8B_tomato | 12.82 |
| 9K_tomato | 21.81 | 9A_tomato | 12.37 | 9B_tomato | 14.75 |
| 10K_tomato | 21.72 | 10A_tomato | 12.39 | 10B_tomato | 13.24 |

Pepper - the results were collected 10 weeks after planting of the seedling: the growth of pepper shoots was observed in the case of Control for all samples (1-10), as well as in the case of Variant A for samples: 2, 4, 5 and 6 and in the case of variant B for samples: 2, 4, 5 and 6. In the variants with the addition of only phytopathogens, low growth was observed, and changes were noted on the leaves (variants A1 and B1, soil with *Pectobacterium carotovorum,* soil with *Pseudomonas syringae,* respectively). In addition to the visual assessment, a quantitative assessment was carried out by weighing the shoots from each of the pots **(Table 11).** Only in the variants A2 and B2, the growth of the shoots at a level comparable to the control sample 1 was observed, which indicates the elimination of pathogenic bacteria as a result of the addition of the biopreparation. In the remaining samples (A 3-10 and B 3-10), where single strains of bacteria were added, the growth was at a lower level as indicated by the biomass distribution in the range of 10.16 - 18.58 and in no case a result at the same level as in the variants A2 and B2 was obtained. In addition, a significant biostimulation of pepper shoots growth was observed in the Control, in the sample 2 - where a mixture of all strains included in the biopreparation was added, the difference compared to the control was 15%, in the other Controls, samples 3-10, no biostimulation was observed, the growth was at the same level as in the Control - sample 1. The obtained results indicate the synergistic effect of strains of bacteria that are included the biopreparation.

**Table 11. Quantitative assessment of the growth of peppers in individual pots. Experiment variants as in the Table 7.**

| Controls | Biomass (g) | Variant A | Biomass (g) | Variant B | Biomass (g) |
|---|---|---|---|---|---|
| 1 K_peppers | 25.84 | 1A_peppers | 14.28 | 1 B_peppers | 13.56 |
| 2K_peppers | 29.76 | 2A_peppers | 26.27 | 2B_peppers | 25.94 |
| 3K_peppers | 23.12 | 3A_peppers | 16.51 | 3B_peppers | 16.19 |
| 4K_peppers | 24.96 | 4A_peppers | 16.79 | 4B_peppers | 16.71 |
| 5K_peppers | 24.58 | 5A_peppers | 18.58 | 5B_peppers | 17.77 |
| 6K_peppers | 25.22 | 6A_peppers | 16.23 | 6B_peppers | 16.12 |
| 7K_peppers | 25.19 | 7A_peppers | 15.89 | 7B_peppers | 16.21 |
| 8K_peppers | 24.68 | 8A_peppers | 10.16 | 8B_peppers | 11.52 |
| 9K_peppers | 24.79 | 9A_peppers | 15.26 | 9B_peppers | 15.54 |
| 10K_peppers | 24.12 | 10A_peppers | 14.45 | 10B_peppers | 15.14 |

### Example 7. Classification of the produced compounds to the group of bacteriocins

To assess whether the compounds produced by the strains of the genus *Bacillus* included in the biopreparation deposited as B/00148 belong to the group of bacteriocins, cross-streak tests were performed. They were carried out on 3 *Bacillus* strains included in the biopreparation: ***Bacillus* sp. strain 202B, *Bacillus* sp. strain 233, *Bacillus* sp. strain 307A.** It was shown that the tested strains do not inhibit each other's growth, that is, they are resistant to the effects of compounds secreted by themselves. This result unambiguously suggests that the tested strains produce bacteriocins, to which they are mutually resistant.

Conclusions from the performed experiments and the obtained results:
a) It was shown **(Example 2)** that between the strains included in the biopreparation composition deposited as B/00148 there is no antagonistic effect, i.e. none of the tested strains changes the environment in such a way as to inhibit the growth of others, thus it has been shown that these strains can be safely co-cultured with each other.
b) It was shown **(Example 3A)** that the strains included in the biopreparation deposited as B/00148: *Bacillus* sp. strain 202B, *Bacillus* sp. strain 233, *Bacillus* sp. strain 307A, A, *Peanibacillus* sp. strain 45, *Pseudomonas* sp. strain 23 (W), *Pseudomonas* sp. strain 60 (W) are capable of degrading organic matter to compounds easily assimilable by plants, being the source of, e.g., C, N and P.
c) It was shown **(Example 3B)** that the strain *Arthrobacter* sp. 1.4. is capable of atmospheric nitrogen binding, thus the administration of the composition according to the invention enables the reduction of the use of nitrogen fertilizers and natural enrichment of the soil with nitrogen compounds that are available to plants.
d) It was shown **(Example 3C)** that the strains of the genera *Bacillus* and *Pseudomonas* included in the biopreparation deposited as B/00148 have properties that are their generic features: amylolytic (decomposition of starch), proteolytic (protein degradation), denitrifying (reduction of nitrates to nitrogen) and ammonifying (conversion of nitrogen contained in organic compounds). Decomposition of organic compounds contributes to the formation of humus and the release of, e.g., phosphorus and nitrogen. Humus determines the fertility of the soil. Thus, the administration of a composition according to the invention enables the formation of humus.
e) It was shown **(Example 4),** that the strains included in the biopreparation B/00148: *Arthrobacter* sp. strain 1.4, *Bacillus* sp. strain 202B, *Bacillus* sp. strain 233, *Bacillus* sp. strain 307A, *Citrobacter* sp. strain 6A, *Peanibacillus* sp. strain 45, *Pseudomonas* sp. strain 23 (W), *Pseudomonas* sp. strain 60 (W) are resistant to Ni(II), Cu(II), Zn(II), As(III), semi-sensitive to Cr(VI) and sensitive to Cd(II). Soil contamination with heavy metals occurs locally in Poland and mainly affects industrial areas. Heavy metals in soils are a potential source of danger for plants - phytotoxic effects as well as for groundwaters, and as a consequence - can be included in the food chain. These elements are taken up and accumulated by vegetables and are harmful to human health. Thus, the administration of the composition according to the invention makes enables inactivation of heavy metals and the performing of other functions of these bacteria even in soils contaminated with heavy metals.
f) It was observed in the plate test **(Example 5),** that the compounds secreted by the strains included in the biopreparation: *Bacillus* sp. strain 202B, *Bacillus* sp. strain 233, *Bacillus* sp. strain 307A, *Citrobacter* sp. strain 6A, *Peanibacillus* sp. strain 45, *Pseudomonas* sp. strain 23 (W), *Pseudomonas* sp. strain 60 (W) completely inhibit the growth of both phytopathogens tested: *Pseudomonas syringae* and *Pectobacterium carotovorum,* thus, it has been shown that the composition according to the invention controls plant pathogens in soil.
g) It was shown **(Example 6)** that the bacteria producing bacteriocins included in the preparation according to the invention can be successfully used as soil preparations with bactericidal properties, as it has been shown in pot experiments that they eliminate bacteria pathogenic to plants from the soil, thus preventing the development of the pathogenesis process in this environment.
h) It was shown **(Example 6)** that the composition according to the invention, after adding to the soil, stimulates plant growth more strongly, as demonstrated on the example of potato, carrot, tomato and pepper, than individual strains included in the biopreparation, by which not only bactericidal activity against phytopathogens: *Pseudomonas syringae* and *Pectobacterium carotovorum,* but also the synergistic effect of the cooperation of the individual bacteria included in the composition according to the invention was demonstrated.
i) It was shown **(Example 7)** that antibacterial compounds produced by the strains of the genus *Bacillus* are bacteriocins.

### Bibliography

1. Ahemad M., Kibret M., 2014, Mechanisms and applications of plant growth promoting rhizobacteria: Current perspective, Journal of King Saud University - Science, 26(1): 1-20.
2. Bender C.L., Alarcón-Chaidez F., Gross D.C., 1999, Pseudomonas syringae Phytotoxins: Mode of Action, Regulation and Biosynthesis by Peptide and Polyketide Synthetases, Microbiol. Mol. Biol. Rev., 63(2): 266-292.
3. Compant S., Duffy B., Nowak J., Clement C., Barka EA. 2005. Use of plant growth-promoting bacteria for biocontrol of plant diseases: principles, mechanisms of action, and future prospects. Appl Environ Microbiol. 71(9):4951-9.
4. Cotter P.D., Hill C., Ross R.P., 2005, Bacteriocins: developing innate immunity for food, Nat. Rev. Microbiol., 3(10): 777-788.
5. Eunjung R., Tae-Ho P., Myung-il K., Seungdon L., Sangryeol R., Chang-Sik O., Sangkee R., Doo-Ho K., Beom-Seok P., Sunggi H, 2010, Characterization of a New Bacteriocin, Carocin D, from Pectobacterium carotovorum subsp. carotovorum Pcc21, Appl. Environ. Microbiol.,76(22): 7541-7549.
6. Glick B.R., 2012, Plant Growth-Promoting Bacteria: Mechanisms and Applications, Scientifica, 15.
7. Hammami R., Fernandez B., Lacroix C., Fliss I., 2013, Anti-infective properties of bacteriocins: an update, Cell. Mol. Life Sci.70(16): 2947-2967.
8. Jastrzebska E, 2010, The Effect of Crop Protection Chemicals on Soil-Dwelling Microorganisms, Contemporary Problems of Management and Environmental Protection, 5:43-53.
9. Lee D.H., Lim J.-A., Lee J., Roh E., Jung K., Choi M., Heu S., 2013, Characterization of genes required for the pathogenicity of Pectobacterium carotovorum subsp. carotovorum Pcc21 in Chinese cabbage, Microbiology, 159(7): 1487-1496.
10. Lo C.C., 2010, Effect of pesticides on soil microbial community, Journal of Environmental Science and Health, Part B: Pesticides, Food Contaminants, and Agricultural Wastes, 45:5, 348-359.
11. Martin-Sanz A., Perez de la Vega M., Murillo J., Caminero C., 2013, Strains of Pseudomonas syringae pv. syringae from Pea Are Phylogenetically and Pathogenically Diverse, Phytopathology, 103(7): 673-681.
12. Riley M.A., and Wertz J. E, 2002, Bacteriocins: Evolution, Ecology, and Application, Annu. Rev. Microbiol., 56: 117-37.
13. Río-Álvarez I., Rodriguez-Herva J. J., Martinez P. M., Gonzalez-Melendi P., Garcia-Casado G., Rodriguez-Palenzuela P., Lopez-Solanilla E., 2013, Light regulates motility, attachment and virulence in the plant pathogen Pseudomonas syringae pv. tomato DC3000, Environ. Microbiol., 16(7): 2072-2085.
14. Sripontan Y., Tan Ch.-W., Hung M.-H., Young Ch.-Ch., Hwang S.-Y. 2014. Effects of plant-growth-promoting microorganisms and fertilizers on growth of cabbage and tomato and Spodoptera litura performance. Journal of Asia-Pacific Entomology 17(3):587-593
15. Subramanian S., Smith D.L., 2015, Bacteriocins from the rhizosphere microbiome - from an agriculture perspective, Front. Plant Sci.,6: 909.
16. Vejan P., Abdullah R., Khadiran T., Ismail S., Nasrulhaq Boyce A., 2016, Role of Plant Growth Promoting Rhizobacteria in Agricultural Sustainability-A Review, Molecules 21(5): 573.
17. Zacharof M.P., Lovitt R.W., 2012, Bacteriocins Produced by Lactic Acid Bacteria a Review Article, APCBEE Procedia, 2: 50-56.

## Claims

1. A composition comprising isolated strains of saprophytic soil bacteria: *Arthrobacter* sp*.* strain 1.4, *Bacillus* sp. strain 202B, *Bacillus* sp. strain 233, *Bacillus* sp. strain 307A, *Citrobacter* sp*.* strain 6A, *Peanibacillus* sp. strain 45, *Pseudomonas* sp. strain 23 (W), *Pseudomonas* sp. strain 60 (W) and deposited under the deposit number B/00148 in the Polish Collection of Microorganisms PCM.

2. A biopreparation comprising the composition of strains as defined in claim 1,wherein the biopreparation is in form of liquid or lyophilisate, or embedded in a carrier.

3. The biopreparation according to claim 2, **characterized in that** additionally contains liquid mineral medium supplemented with glucose as the sole carbon source or solid mineral medium supplemented with glucose as the sole carbon source.

4. The biopreparation according to claims 2-3, **characterized in that** contains at least 10⁵ bacterial cells per ml of the medium, more preferably at least 10⁶ bacterial cells per ml of the medium, more preferably the cfu of each strain is at least 10⁶ per ml of the medium,
and wherein the strains *Arthrobacter* sp. strain 1.4, *Bacillus* sp. strain 202B, *Bacillus* sp. strain 233, *Bacillus* sp. strain 307A, *Citrobacter* sp. strain 6A, *Peanibacillus* sp. strain 45, *Pseudomonas* sp. strain 23 (W), *Pseudomonas* sp. strain 60 (W) are in a substantially equal quantitative ratio to each other.

5. A method of production of the composition as defined in claim 1 and/or biopreparation as defined in claims 2-4, **characterized in that** the culturing of strains *Arthrobacter* sp. strain 1.4, *Bacillus* sp. strain 202B, *Bacillus* sp. strain 233, *Bacillus* sp. strain 307A, *Citrobacter* sp. strain 6A, *Peanibacillus* sp. strain 45, *Pseudomonas* sp. strain 23 (W), *Pseudomonas* sp. strain 60 (W) deposited under the deposit number B/00148 in the Polish Collection of Microorganisms PCM, is carried out
**a)** simultaneously in a medium providing the growth of each of the strains, or
**b)** each of the strains is cultured individually in an appropriate medium enabling its growth, and the obtained cultures are mixed in order to obtain the biopreparation, preferably each strain is mixed with the other in a substantially equal quantitative ratio,
wherein, preferably, the culturing in **a)** and/or **b)** is carried out at a temperature in the range of 20-25°C,
wherein, preferably, the medium use in the step of culturing **a)** and/or **b)** is liquid mineral medium supplemented with glucose as the sole carbon source or solid mineral medium supplemented with glucose as the sole carbon source.

6. A method of plant pathogen control in soil comprising the step of introducing of the composition as defined in claim 1 and/or biopreparation as defined in claims 2-4 and/or composition and/or biopreparation produced by the method as defined in claim 5, into the ground,
preferably wherein, the introduction into the ground is carried out by sprinkling,
preferably wherein, the method additionally comprises the steps - mechanical oxygenation of soil and humidification of soil to maintain the appropriate soil moisture level.

7. The method of plant pathogen control in soil according to claim 6, **characterized in that** the pathogen is selected from *Pseudomonas syringae, Pectobacterium carotovorum,* wherein
more preferably *Pseudomonas syringae* is a pathogen of tomato, peas, cucumber, soy, cauliflower, beans and fruit trees, potato, carrot, pepper, and/or
more preferably *Pectobacterium carotovorum* is a pathogen of carrot, potato, tomato, pepper, green leafy vegetables, pumpkin, onion,
wherein, preferably the method takes place *ex situ* or takes place *in situ.*

8. The method of plant pathogen control in soil according to claims 6-7, **characterized in that** the soil is sprinkled with the biopreparation in the amount of 10 liters per 1 ha of land surface and/or the biopreparation is introduced into the ground in a volume ratio biopreparation: soil in the range from 1:10 to 1:30, wherein the initial preparation used for sprinkling and/or introducing into the ground is preferably diluted in a ratio 1:50 to 1:100.

9. A method of soil fertilization and restoring the natural biological balance of soil microflora comprising the step of introducing of the composition defined in claim 1 and/or biopreparation defined in claims 2-4 and/or produced by the method defined in claim 5, into the ground,
wherein preferably the introduction into the ground is carried out by sprinkling,
preferably after intensive use of chemical plant protection products,
wherein the method preferably takes place *ex situ* or takes place *in situ.*

10. The method of soil fertilization and restoring the natural biological balance of soil microflora, according to claim 9 , **characterized in that** the biopreparation is introduced into the ground in the amount of 10 liters per 1 ha of land surface and/or in a volume ratio biopreparation: soil in the range from 1:10 to 1:30, wherein the initial preparation used for sprinkling and/or introducing into the ground is preferably diluted in a ratio 1:50 to 1:100.

11. A method of biostimulation of plant development and growth comprising the step of introducing of the composition defined in claim 1 and/or biopreparation defined in claims 2-4 and/or produced by the method defined in claim 5, into the ground, wherein preferably the introduction into the ground is carried out by sprinkling,
wherein, preferably, the method takes place *ex situ* or takes place *in situ,*
wherein, preferably, the plants are selected from tomato, peas, cucumber, soy, cauliflower, beans and fruit trees, carrot, potato, tomato, green leafy vegetables, pumpkin, onion, pepper.

12. The method of biostimulation of plant development and growth according to claim 11, **characterized in that** the biopreparation is introduced into the ground in the form of diluted preparation in the amount of 10 liters per 1 ha of land surface and/or in a volume ratio biopreparation: soil in the range from 1:10 to 1:30, wherein the initial preparation used for sprinkling and/or introducing into the ground is preferably diluted in a ratio 1:50 to 1:100.

13. Use of the composition defined in claim 1 and/or biopreparation defined in claims 2-4 and/or produced by the method defined in claim 5 for plant pathogen control in soil, preferably wherein, the pathogen is selected from *Pseudomonas syringae, Pectobacterium carotovorum,*
more preferably *Pseudomonas syringae* is a pathogen of tomato, peas, cucumber, soy, cauliflower, beans and fruit trees, potato, carrot, pepper, and/or
more preferably *Pectobacterium carotovorum* is a pathogen of carrot, potato, tomato, pepper, green leafy vegetables, pumpkin, onion.

14. Use of the composition defined in claim 1 and/or biopreparation defined in claims 2-4 and/or produced by the method defined in claim 5 for soil fertilization and restoring the natural biological balance of soil microflora, preferably after intensive use of chemical plant protection products.

15. Use of the composition defined in claim 1 and/or biopreparation defined in claims 2-4 and/or produced by the method defined in claim 5 for biostimulation of plant development and growth, preferably wherein the plants are selected from tomato, peas, cucumber, soy, cauliflower, beans and fruit trees, carrot, potato, pepper, green leafy vegetables, pumpkin, onion.

## Patentansprüche

1. Eine Zusammensetzung, die isolierte Stämme saprophytischer Bodenbakterien enthält: *Arthrobacter sp.* Stamm 1.4, *Bacillus sp.* Stamm 202B, *Bacillus sp.* Stamm 233, *Bacillus sp.* Stamm 307A, *Citrobacter sp.* Stamm 6A, *Peanibacillus sp.* Stamm 45, *Pseudomonas sp.* Stamm 23 (W), *Pseudomonas sp.* Stamm 60 (W) und hinterlegt unter der Hinterlegungsnummer B/00148 in der Polnischen Sammlung von Mikroorganismen PKM.

2. Ein Biopräparat, umfassend die Zusammensetzung von Stämmen wie in Anspruch 1 definiert, wobei das Biopräparat in Form von Flüssigkeit oder Lyophilisat oder eingebettet in einen Träger vorliegt.

3. Das Biopräparat nach Anspruch 2, **dadurch gekennzeichnet, dass** es zusätzlich flüssiges mineralisches Medium, das mit Glucose als einziger Kohlenstoffquelle ergänzt ist, oder festes mineralisches Medium, das mit Glucose als einziger Kohlenstoffquelle ergänzt ist, enthält.

4. Biopräparat nach den Ansprüchen 2-3, **dadurch gekennzeichnet, dass** es mindestens 10⁵ Bakterienzellen pro ml des Mediums enthält, bevorzugter mindestens 10⁶ Bakterienzellen pro ml des Mediums, bevorzugter die KbE jedes Stammes mindestens 10⁶ pro ml des Mediums beträgt,
und wobei die Stämme *Arthrobacter* sp. Stamm 1.4, *Bacillus* sp. Stamm 202B, *Bacillus* sp. Stamm 233, *Bacillus* sp. Stamm 307A, *Citrobacter* sp. Stamm 6A, *Peanibacillus* sp. Stamm 45, *Pseudomonas* sp. Stamm 23 (W), *Pseudomonas* sp. Stamm 60 (W) in einem im wesentlichen gleichen quantitativen Verhältnis zueinander stehen.

5. Ein Verfahren zur Herstellung der Zusammensetzung nach Anspruch 1 und/oder eines Biopräparats nach den Ansprüchen 2-4, **dadurch gekennzeichnet, dass** die Kultivierung der Stämme *Arthrobacter* sp. Stamm *1.4, Bacillus* sp. Stamm 202B, *Bacillus* sp. Stamm 233, *Bacillus* sp. Stamm 307A, *Citrobacter* sp. Stamm 6A, *Peanibacillus* sp. Stamm 45, *Pseudomonas* sp. Stamm 23 (W), *Pseudomonas* sp. Stamm 60 (W), hinterlegt unter der Hinterlegungsnummer B/00148 in der Polnischen Sammlung von Mikroorganismen PKM, wird
a) gleichzeitig in einem Medium durchgeführt, das das Wachstum jedes der Stämme ermöglicht, oder
b) jeder der Stämme wird einzeln in einem geeigneten Medium kultiviert, das sein Wachstum ermöglicht, und die erhaltenen Kulturen werden gemischt, um das Biopräparat zu erhalten, wobei vorzugsweise jeder Stamm mit dem anderen in einem im wesentlichen gleichen quantitativen Verhältnis gemischt wird,
wobei vorzugsweise die Kultivierung in a) und/oder b) bei einer Temperatur im Bereich von 20-25°C durchgeführt wird,
wobei vorzugsweise das in der Stufe der Kultivierung von a) und/oder b) verwendete Medium ein flüssiges mineralisches Medium ist, das mit Glucose als einziger Kohlenstoffquelle ergänzt ist, oder ein festes mineralisches Medium, das mit Glucose als einziger Kohlenstoffquelle ergänzt ist.

6. Ein Verfahren zur Bekämpfung von Pflanzenkrankheitserregern im Boden, umfassend den Schritt des Einbringens der Zusammensetzung nach Anspruch 1 und/oder eines Biopräparats nach Anspruch 2-4 und/oder einer Zusammensetzung und/oder eines Biopräparats, die durch das Verfahren nach Anspruch 5 hergestellt wurden, in den Boden,
wobei das Einbringen in den Boden vorzugsweise durch Berieselung erfolgt,
wobei das Verfahren zusätzlich die Schritte - mechanische Sauerstoffanreicherung des Bodens und Befeuchtung des Bodens zur Aufrechterhaltung des geeigneten Bodenfeuchtigkeitsniveaus umfasst.

7. Das Verfahren zur Bekämpfung von Pflanzenkrankheitserregernim Boden nach Anspruch 6, **dadurch gekennzeichnet, dass** der Erreger aus *Pseudomonas syringae, Pectobacterium carotovorum* ausgewählt ist, wobei
noch bevorzugter *Pseudomonas syringae* ist ein Krankenheitserreger von Tomaten, Erbsen, Gurken, Soja, Blumenkohl, Bohnen und Obstbäumen, Kartoffeln, Karotten, Paprika, und/oder
noch bevorzugter, *Pectobacterium carotovorum* ist ein Krankenheitserreger von Karotten, Kartoffeln, Tomaten, Paprika, grünem Blattgemüse, Kürbis, Zwiebeln,
wobei das Verfahren vorzugsweise *ex situ* oder *in situ* stattfindet.

8. Das Verfahren zur Bekämpfung von Pflanzenkrankheitserregern im Boden nach den Ansprüchen 6-7, **dadurch gekennzeichnet, dass** der Boden mit dem Biopräparat in einer Menge von 10 Litern pro 1 ha Landoberfläche berieselt wird und/oder das Biopräparat in einem Volumenverhältnis Biopräparat: Boden im Bereich von 1:10 bis 1:30 in den Boden eingebracht wird, wobei das zur Berieselung und/oder Einbringen in den Boden verwendete Ausgangspräparat vorzugsweise in einem Verhältnis von 1:50 bis 1:100 verdünnt wird.

9. Ein Verfahren zur Bodendüngung und Wiederherstellung des natürlichen biologischen Gleichgewichts der Bodenmikroflora, umfassend den Schritt des Einbringens der in Anspruch 1 definierten Zusammensetzung und/oder des in den Ansprüchen 2-4 definierten Biopräparats und/oder des nach dem in Anspruch 5 definierten Verfahren hergestellten Biopräparats in den Boden,
wobei das Einbringen in den Boden vorzugsweise durch Berieselung,
vorzugsweise nach intensiver Verwendung von chemischen Pflanzenschutzmitteln, erfolgt,
wobei das Verfahren vorzugsweise *ex situ* oder *in situ* stattfindet.

10. Das Verfahren zur Bodendüngung und zur Wiederherstellung des natürlichen biologischen Gleichgewichts der Bodenmikroflora nach Anspruch 9, **dadurch gekennzeichnet, dass** das Biopräparat in den Boden in einer Menge von 10 Litern pro 1 ha Landoberfläche und/oder in einem Volumenverhältnis Biopräparat: Boden im Bereich von 1:10 bis 1:30 eingebracht wird, wobei das zur Berieselung und/oder Einbringen in den Boden verwendete Ausgangspräparat vorzugsweise in einem Verhältnis von 1:50 bis 1:100 verdünnt wird.

11. Ein Verfahren zur Biostimulation der Pflanzenentwicklung und des Pflanzenwachstums, umfassend den Schritt des Einbringens der in Anspruch 1 definierten Zusammensetzung und/oder des in den Ansprüchen 2-4 definierten Biopräparats und/oder des nach dem in Anspruch 5 definierten Verfahren hergestellten Biopräparats in den Boden, wobei das Einbringen in den Boden vorzugsweise durch Berieselung durchgeführt wird,
wobei das Verfahren vorzugsweise *ex situ* oder *in situ* stattfindet,
wobei die Pflanzen vorzugsweise ausgewählt sind aus Tomaten, Erbsen, Gurken, Soja, Blumenkohl, Bohnen und Obstbäumen, Karotten, Kartoffeln, Tomaten, grünem Blattgemüse, Kürbis, Zwiebeln, Paprika.

12. Das Verfahren zur Biostimulation der Pflanzenentwicklung und des Pflanzenwachstums nach Anspruch 11, **dadurch gekennzeichnet, dass** das Biopräparat in Form eines verdünnten Präparats in der Menge von 10 Litern pro 1 ha Landoberfläche und/oder in einem Volumenverhältnis Biopräparat: Boden im Bereich von 1:10 bis 1:30 in den Boden eingebracht wird, wobei das zur Berieselung und/oder Einbringen in den Boden verwendete Ausgangspräparat vorzugsweise in einem Verhältnis von 1:50 bis 1:100 verdünnt wird.

13. Verwendung der Zusammensetzung nach Anspruch 1 und/oder eines Biopräparats nach den Ansprüchen 2-4 und/oder hergestellt nach dem Verfahren nach Anspruch 5 zur Bekämpfung von Pflanzenkrankheitserregern im Boden, wobei der Krankenheitserreger vorzugsweise aus *Pseudomonas syringae, Pectobacterium carotovorum* ausgewählt ist,
noch bevorzugter *Pseudomonas syringae* ist ein Erreger von Tomaten, Erbsen, Gurken, Soja, Blumenkohl, Bohnen und Obstbäumen, Kartoffeln, Karotten, Paprika, und/oder
bevorzugter *Pectobacterium carotovorum* ist ein Erreger von Karotten, Kartoffeln, Tomaten, Paprika, grünem Blattgemüse, Kürbis, Zwiebeln.

14. Verwendung der in Anspruch 1 definierten Zusammensetzung und/oder des in den Ansprüchen 2-4 definierten Biopräparats und/oder des nach dem in Anspruch 5 definierten Verfahren hergestellten Biopräparats zur Bodendüngung und Wiederherstellung des natürlichen biologischen Gleichgewichts der Bodenmikroflora, vorzugsweise nach intensivem Einsatz von chemischen Pflanzenschutzmitteln.

15. Verwendung der in Anspruch 1 definierten Zusammensetzung und/oder des in den Ansprüchen 2-4 definierten Biopräparats und/oder hergestellt nach dem in Anspruch 5 definierten Verfahren zur Biostimulation der Pflanzenentwicklung und des Pflanzenwachstums, wobei die Pflanzen vorzugsweise aus Tomaten, Erbsen, Gurken, Soja, Blumenkohl, Bohnen und Obstbäumen, Karotten, Kartoffeln, Paprika, grünem Blattgemüse, Kürbis, Zwiebeln ausgewählt sind.

## Revendications

1. Une composition comprenant des souches isolées de bactéries saprophytes du sol : la souche 1.4 de Arthrobacter sp., la souche 202B de Bacillus sp., la souche 233 de Bacillus sp., la souche 307A de Bacillus sp., la souche 6A de Citrobacter sp., la souche 45 de Peanibacillus sp., la souche 23 (W) de Pseudomonas sp., la souche 60 (W) de Pseudomonas sp. et déposée sous le numéro de dépôt B/00148 dans la Collection polonaise de micro-organismes PCM.

2. Une biopréparation comprenant la composition de souches telle que définie dans la revendication 1, où la biopréparation est sous forme de liquide ou de lyophilisat, ou noyée dans un support.

3. La préparation biologique selon la revendication 2, **caractérisée en ce qu'**elle contient en outre un milieu minéral liquide complété avec du glucose comme seule source de carbone ou un milieu minéral solide complété avec du glucose comme seule source de carbone.

4. La biopréparation selon les revendications 2-3, **caractérisée en ce qu'**elle contient au moins 10⁵ cellules bactériennes par ml du milieu, plus préférentiellement au moins 10⁶ cellules bactériennes par ml du milieu, plus préférablement le CFU de chaque souche est d'au moins 10⁶ par ml du milieu,
et où la souche 1.4 d'Arthrobacter sp., la souche 202B de Bacillus sp., la souche 233 de Bacillus sp., la souche 307A de Bacillus sp., la souche 6A de Citrobacter sp., la souche 45 de Peanibacillus sp., la souche 23 (W) de Pseudomonas sp., la souche 60 (W) de Pseudomonas sp. sont dans un rapport quantitatif sensiblement égal entre elles.

5. Une méthode de production de la composition selon la revendication 1 et/ou de la biopréparation selon les revendications 2 à 4, **caractérisée en ce que** la culture de souches de la souche 1.4 d'Arthrobacter sp. , la souche 202B de Bacillus sp., la souche 233 de Bacillus sp., la souche 307A de Bacillus sp., la souche 6A de Citrobacter sp., la souche 45 de Peanibacillus sp., la souche 23 (W) de Pseudomonas sp., la souche 60 (W) de Pseudomonas sp. déposée sous le numéro de dépôt B/00148 dans la Collection polonaise de micro-organismes PCM, est réalisée
**a)** simultanément dans un milieu assurant la croissance de chacune des souches, ou
**b)** chacune des souches est cultivée individuellement dans un milieu approprié permettant sa croissance, et les cultures obtenues sont mélangées afin d'obtenir la biopréparation, de préférence chaque souche est mélangée avec l'autre dans un rapport quantitatif sensiblement égal,
où de préférence la culture en **a)** et/ou **b)** est réalisée à une température dans la plage de 20 à 25°C, où de préférence le milieu utilisé dans l'étape de culture **a)** et/ou **b)** est un milieu minéral liquide complété avec du glucose comme seule source de carbone ou un milieu minéral solide complété avec du glucose comme seule source de carbone.

6. Une méthode de lutte contre les pathogènes des plantes dans le sol comprenant l'étape d'introduction de la composition telle que définie dans la revendication 1 et/ou de la biopréparation telle que définie dans les revendications 2 à 4 et/ou de la composition et/ou de la biopréparation produite par la méthode telle que définie dans la revendication 5, dans le sol,
où de préférence l'introduction dans le sol est réalisée par aspersion,
où de préférence la méthode comprend en outre les étapes d'oxygénation mécanique du sol et d'humidification du sol pour maintenir le niveau d'humidité du sol approprié.

7. La méthode de lutte contre les pathogènes des plantes dans le sol selon la revendication 6, **caractérisée en ce que** le pathogène est choisi parmi *Pseudomonas syringae, Pectobacterium carotovorum,* où
plus préférablement *Pseudomonas syringae* est un pathogène de la tomate, des pois, du concombre, du soja, du chou-fleur, des haricots et des arbres fruitiers, de la pomme de terre, de la carotte, du poivre et/ou
plus préférablement *Pectobacterium carotovorum* est un pathogène de la carotte, de la pomme de terre, de la tomate, du poivron, des légumes à feuilles vertes, de la citrouille, de l'oignon,
où de préférence la méthode a lieu *ex situ* ou a lieu *in situ.*

8. La méthode de lutte contre les pathogènes des plantes dans le sol selon les revendications 6 à 7, **caractérisée en ce que** le sol est aspergé avec le biopréparation à raison de 10 litres par 1 ha de surface de terre et/ou la biopréparation est introduite dans le sol dans un rapport volumique biopréparation : sol dans la plage de 1:10 à 1:30, où la préparation initiale utilisée pour l'arrosage et/ou l'introduction dans le sol est de préférence diluée dans un rapport de 1:50 à 1:100.

9. Une méthode de fertilisation du sol et de restauration de l'équilibre biologique naturel de la microflore du sol comprenant l'étape d'introduction de la composition définie dans la revendication 1 et/ou de la biopréparation définie dans les revendications 2 à 4 et/ou produite par la méthode définie dans la revendication 5, dans le sol,
où de préférence l'introduction dans le sol est réalisée par aspersion,
de préférence après une utilisation intensive de produits phytosanitaires chimiques,
où de préférence la méthode a lieu *ex situ* ou a lieu *in situ.*

10. La méthode de fertilisation du sol et de restauration de l'équilibre biologique naturel de la microflore du sol, selon la revendication 9, **caractérisée en ce que** la biopréparation est introduite dans le sol à raison de 10 litres par 1 ha de surface de terre et/ou dans un rapport volumique biopréparation : sol dans la plage de 1:10 à 1:30, où la préparation initiale utilisée pour l'arrosage et/ou l'introduction dans le sol est de préférence diluée dans un rapport de 1:50 à 1:100.

11. Une méthode de biostimulation du développement et de la croissance des plantes comprenant l'étape d'introduction de la composition définie dans la revendication 1 et/ou de la biopréparation définie dans les revendications 2 à 4 et/ou produite par la méthode définie dans la revendication 5, dans le sol, où de préférence l'introduction dans le sol est réalisée par aspersion,
où de préférence la méthode a lieu *ex situ* ou a lieu *in situ.*
où de préférence les plantes sont choisies parmi la tomate, les pois, le concombre, le soja, le chou-fleur, les haricots et les arbres fruitiers, la carotte, la pomme de terre, la tomate, les légumes à feuilles vertes, la citrouille, l'oignon, le poivron.

12. La méthode de biostimulation du développement et de la croissance des plantes selon la revendication 11, **caractérisée en ce que** la biopréparation est introduite dans le sol sous forme de préparation diluée à raison de 10 litres par 1 ha de surface de terre et/ou dans un rapport volumique biopréparation : sol dans la plage de 1:10 à 1:30, où la préparation initiale utilisée pour l'arrosage et/ou l'introduction dans le sol est de préférence diluée dans un rapport de 1:50 à 1:100.

13. Utilisation de la composition définie dans la revendication 1 et/ou de la biopréparation définie dans les revendications 2 à 4 et/ou produite par la méthode définie dans la revendication 5 pour la lutte contre les pathogènes des plantes dans le sol, où de préférence le pathogène est choisi parmi *Pseudomonas syringae, Pectobacterium carotovorum,*
plus préférablement *Pseudomonas syringae* est un pathogène de la tomate, des pois, du concombre, du soja, du chou-fleur, des haricots et des arbres fruitiers, de la pomme de terre, de la carotte, du poivre et/ou
plus préférablement *Pectobacterium carotovorum* est un pathogène de la carotte, de la pomme de terre, de la tomate, du poivron, des légumes à feuilles vertes, de la citrouille, de l'oignon.

14. Utilisation de la composition définie dans la revendication 1 et/ou de la biopréparation définie dans les revendications 2 à 4 et/ou produite par la méthode définie dans la revendication 5 pour la fertilisation du sol et la restauration de l'équilibre biologique naturel de la microflore du sol, de préférence après une utilisation intensive de produits phytosanitaires chimiques.

15. Utilisation de la composition définie dans la revendication 1 et/ou de la biopréparation définie dans les revendications 2 à 4 et/ou produite par la méthode définie dans la revendication 5 pour la biostimulation du développement et de la croissance des plantes, où de préférence les plantes sont choisies parmi la tomate, les pois, le concombre, le soja, le chou-fleur, les haricots et les arbres fruitiers, la carotte, la pomme de terre, le poivron, les légumes à feuilles vertes, la citrouille, l'oignon.
